# EUROPEAN PATENT SPECIFICATION

(11) **EP 0 926 156 B1**
(45) Date of publication and mention of the grant of the patent: **21.09.2005**
(21) Application number: 98124375.1
(22) Date of filing: 22.12.1998
(51) Int. Cl.: C07K 14/35, C12N 15/86, A61K 39/04, A61K 48/00

(54) **Vector comprising Mycobacterium tuberculosis 38 kDa antigen for neoplasm treatment**
Vektor enthaltend Mycobacterium Tuberculosis 38 kDa Antigen zur Behandlung von Neoplasmen
Vecteur comportant l'antigène de 38 kDa de Mycobacterium tuberculosis pour le traitement des néoplasmas

(30) Priority: 22.12.1997 EP 97122698
(43) Date of publication of application: 30.06.1999
(73) Proprietor: Lionex GmbH, 38124 Braunschweig (DE)
(72) Inventor: Singh, Mahavir, Dr., 38124 Braunschweig (DE); Colnaghi, Maria I., 38124 Braunschweig (DE); Colombo, Mario P., 38124 Braunschweig (DE); Balsari, Andrea, 38124 Braunschweig (DE)
(74) Representative: Kröncke, Rolf

(56) References cited:
- ZHU, X. ET AL.: "Specificity of CD8+ T cells from subunit-vaccinated and infected H-2b mice recognizing the 38 kDa antigen of Mycobacterium tuberculosis" INTERNATIONAL IMMUNOLOGY, vol. 9, no. 11, November 1997 (1997-11), pages 1669-1676, XP000925937
- SFONDRINI, L. ET AL.: "Anti-tumor immunity induced by murine melanoma cells transduced with the Mycobacterium tuberculosis gene encoding the 38-kDa antigen" GENE THERAPY, vol. 5, no. 2, February 1998 (1998-02), pages 247-252, XP000925962
- LUKACS, K.V. ET AL.: "Tumor Cells Transfected with a Bacterial Heat-Shock Gene Lose Tumorigenicity and Induce Protection against Tumors" JOURNAL OF EXPERIMENTAL MEDICINE, vol. 178, no. 1, July 1993 (1993-07), pages 343-348, XP000615289
- JONES, V.E. & MITCHELL, M.S.: "Therapeutic vaccines for melanoma: progress and problems" TRENDS IN BIOTECHNOLOGY, vol. 14, no. 9, 1 September 1996 (1996-09-01), pages 349-355, XP004035740

## Description

Recent studies have identified cell surface antigens on murine and human tumors of various histotypes that are potential targets for an immune response. These antigens, which are recognizedy host T cells, derive from endogenously synthesized proteins that arise either through mutations during malignant transformation or through aberrant expression of genes normally silent in adult tissues.¹ However, these antigens are usually very weak immunogens in spontaneously arising tumors. In efforts to elicit an immune response against tumor antigens, various micro-organisms, especially Mycobacterium bovis bacillus Calmette-Guérin (BCG), have been used as immunoadjuvants in patients with different cancer types,
but the results have been variable.² At present, patients with superficial bladder cancer are being treated with immunotherapy consisting of intravesical instillation of BCG. There is some evidence that a similar mechanism underlies the efficacy of BCG in tumor prevention and therapy ³ and in prophylaxis against tuberculosis. This efficacy appears to depend on whether a predominant Th1- or a Th2-type cytokine secretion pattern is induced.²
In another approach to stimulating an immune response against tumors, different cytokine genes have been introduced into tumor cells. The secreted cytokines have been shown to induce a protective immune reaction against the tumor.⁴ For example, administration of engineered tumor cells expressing interleukin-2 (IL-2), IL-4, interferon-gamma (IFN-γ) or granulocyte-macrophage colony-stimulating factor (GM-CSF) has been shown to induce local and often systemic immunity, though elimination of pre-existing tumors was less frequently achieved.⁵ However, in some cases, cytokine-engineered tumor cells reportedly display an increased metastatic potential ⁶.

Further, prior art ¹⁸ discloses the transformation of the murine macrophage tumour cell line J774 with the highly immunogenic hsp 65 from Mycobacterium leprae and concludes the loss of tumourigenicity is achieved by this procedure through enhancement of recognition of tumour-associated antigens. Further, reference is made to the transformation ²⁷ of mouse tumour cell lines, namely the thymoma line EL4 and the lymphoma line RMA with a plasmid encoding Ag38, pXJ38. However, transformation of these cells with Ag38 merely served the purpose of creating cells useful for induction of lymphocytotoxic response against tubercle bacilli upon mice inoculation and there is no indication whatsoever to use vectors according Ag38 for treatment of neoplasms. Finally, reference is made to a revue²⁸ on therapeutic vaccines for melanoma, wherein the revue concerns also vaccines with BCG.

Because the combination of poorly immunogenic and highly immunogenic determinants on a common carrier can augment the immune response toward the poorly antigenic determinants by stimulating immune recognition,⁷ the invention started from the question whether the immune recognition of malignant cells might be enhanced by transducing tumor cells with a gene that encodes a mycobacterium protein, that should represent a danger signal, since it induces a strong T-cell response.⁸ If the expression of this gene leads to enhanced Th1 cell maturation, a T-cell response might also be induced against the tumor cell antigens and eventually result in the selective destruction of tumor cells through a specific anti-tumor immune responxe. According to the present invention, the murine melanoma cell line B16-B78 was trancduced with a Mycobacterium tuberculosis (strain H37Rv) gene encoding a 38-kDa protein (Ag38), ⁹ which is one of the most immunogenic of the Mycobacterium tuberculosis antigens. ¹⁰ Transduced cells were injected into mice and the immune response against parental B16-B78 melanoma cells was evaluated.

As regards the known 38 kDa antigen of *Mycobacterium tuberculosis* and homologues thereof reference can be made for example to EP-A-92 108 254.1 and references cited therein.

The expression vector according to the invention may be a vector wherein the polypeptide is the antigen of M. tuberculosis of 33 or 38 kDa, each wild-type or recombinant.

The expression vector according to the invention may be a virus, especially a disabled retro virus, a retroviral shuttle vector, such as pLTMSN, a vaccina virus, an adeno and/or a plasmid, and/or the nucleic acid may be RNA.

Further, the expression vector according to the invention can be an episomal vector.

Further, the expression vector according to the invention can include a sequence which encodes a neoplasm associated antigen an/or a sequence which encodes an interleukin.

According to another embodiment the invention concerns the use of a cell, especially a neoplasm cell line, especially a human cell or cell line, which includes an expression vector according to the invention.

Further, the invention concerns a cell or virus having in ist genome heterologous nucleic acid encoding a polypeptide as defined above in expressible form.

A cell or cell line according to the invention may further include an expression vector having a sequence which encodes a neoplasm accociated antigen.

A cell line or a cell according to the invention may further include or comprise an expression vector having a sequence which encodes a neoplasm associated antigen or an interleukin.

Another ebodiment of the invention concerns the use for the preparation of a pharmaceutical composition comprising the expression vector, cell, cell line or virus according to the invention and a pharmaceutically acceptable carrier such as a liposome.

The pharmaceutical composition may further comprise an expression vector having a sequence which encodes a neoplasm associated antigen or an interleukin.

The pharmaceutical composition may comprise an effective non-toxic amount of the expression vector, cell line, cell or virus as described above.

According to the invention the expression vector, cell line, cell, virus or nucleic acid are used for the manufacture of a medicament for the treatment of neoplasms, wherein the treatment can be of existing neoplasms or prophylactic.

A discussion of the embodiments of the invention follows.

### Tumorigenicity of transduced melanoma cells

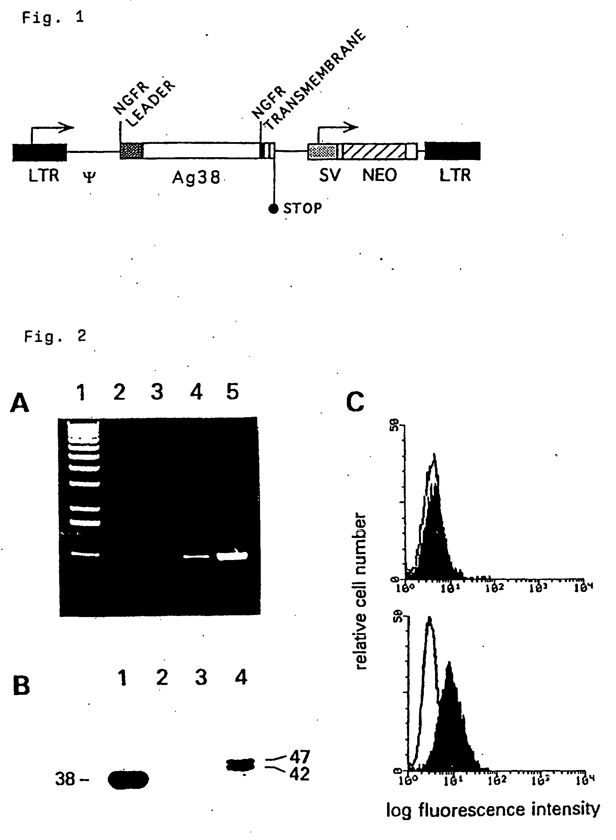

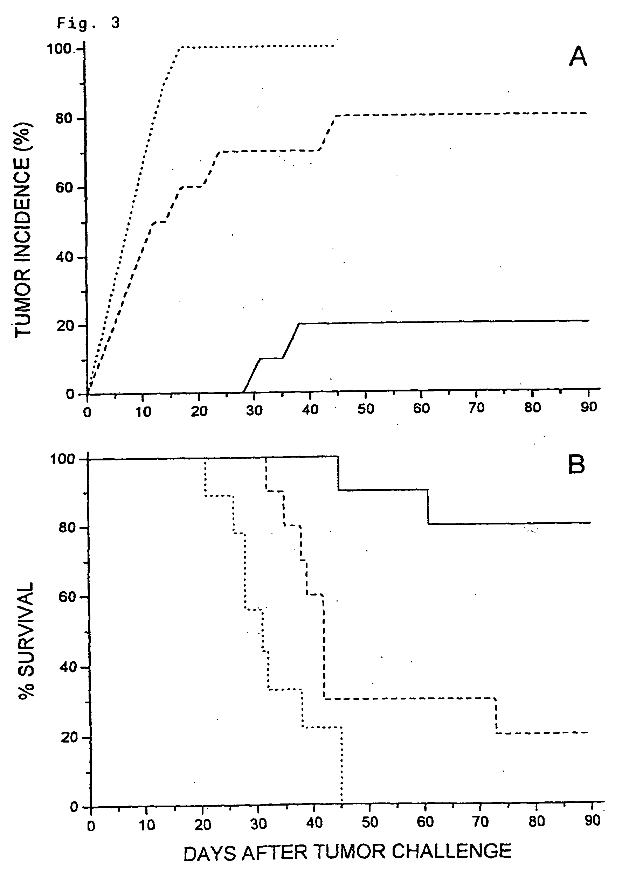

A modified retroviral vector (pLTMSN) containing the leader and transmembrane portion of the human nerve growth factor receptor (NGFR) and the stop codon region of human IL-3 was constructed. The DNA encoding residues 24-372 of Mycobacterium tuberculosisAg38 gene, excluding the leader peptide and the stop codon, was cloned in-frame to obtain the pLAg38TMSN vector (Fig. 1). This modified vector expresses the 38-kDa antigen on the cell surface, allowing detection and tagging of transduced cells by an anti-Ag38 monoclonal antibody (MAb), HBT12. ⁹ The supernatant of the producer cell lines was tested for viral titer and antigen expression by FACScan analysis with MAb HBT12 and used for infection of B16-B78 murine melanoma cells. ¹¹ Transduced cells expressed the Mycobacterium tuberculosis Ag38 transcript and produced the bacterial protein as detected by RT-PCR (Fig. 2A) and Western blot (Fig. 2B), respectively. Reactivity with MAb HBT12 revealed two bands in the total cell membrane extract at 47 kDa and 42 kDa, corresponding, respectively, to the unprocessed form, which contains both the leader and transmembrane sequences, and to the mature protein after cleavage of the leader sequence. The majority of transduced B16-B78 cells were positive for cell surface expression of the mycobacterial protein as determined by FACS analysis (Fig. 2C). Both wild-type and melanoma cells transduced with the vector lacking the Ag38 gene showed no detectable mycobacterial product by RT-PCR, Western blot or FACS analysis.

The tumorigenicity of the parental melanoma cell line and of the cells transduced with the 38-kDa gene was tested in C57BL/6 mice. All 5 mice injected s.c. with 2.5x10⁵ B16-B78 cells developed tumors within 9 to 21 days (mean 13.8) and had a mean survival time of 36.6 days. Necroscopy revealed lung metastases in all of these mice. By contrast, 3 out of 5 mice injected with 2.5x10⁵ transduced melanoma cells developed tumors and these mice showed a delay in appearance of the tumor (mean 30.6 days) that resulted in an increase in survival time (mean 52.3 days); no metastases were observed in the lungs, liver, kidneys or peritoneal cavity. Two other 38-kDa transduced clones, obtained in independent transductions, also showed no spontaneous metastasis (not shown), making it unlikely that the Ag38 gene was inserted into a gene involved in metastasis.

### Ag38-transduced melanoma cells confer systemic immunity against parental melanoma cells

In experiments to determine whether immunization with transduced melanoma cells protected mice against a subsequent challenge with parental cells (Fig. 3), all of 9 naive mice and 8 out of 10 mice immunized with wild-type melanoma cells developed tumors, whereas significant protection (p<0.01) was observed in mice immunized with the transduced melanoma cells, since only 2 out of 10 mice developed tumors. Moreover, in these mice, the onset of tumor development as well as the mortality were delayed in comparison with non-immune and wild-type melanoma-immunized animals. Since wild-type melanoma cells are highly tumorigenic and poorly immunogenic, these data show that the expression of the 38-kDa antigen in transduced cells elicits a strong immune response against the tumor.
Endogenous expression of Ag38 appears to be essential for protection, since C57BL/6 mice immunized with the purified 38-kDa protein developed tumors at the same rate as non-immunized controls when challenged with wild-type melanoma cells.

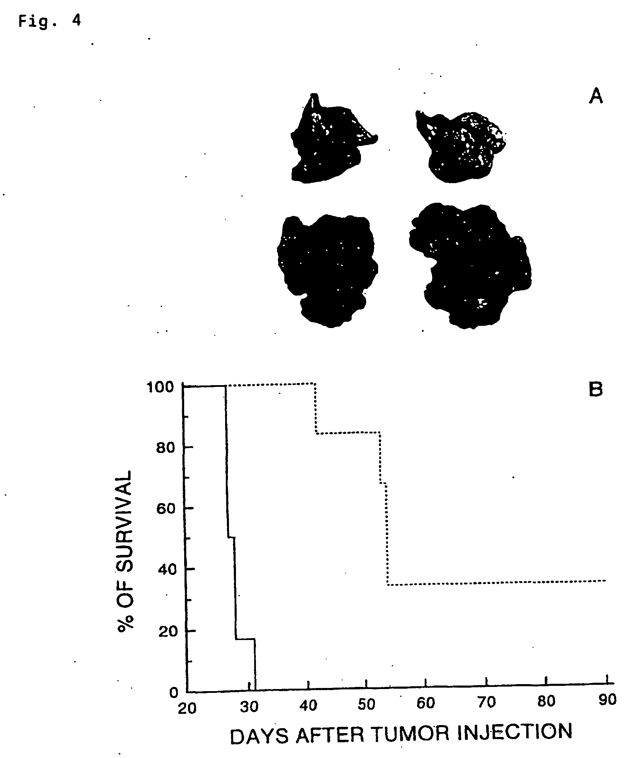

Experimental metastases induced by i.v. inoculation with 5x10⁵ wild-type melanoma cells were inhibited in mice immunized with irradiated Ag38-transduced cells as indicated by the absence or the low number of lung metastases at 4 weeks after challenge, as compared to control mice (Fig. 4A). Two experiments were performed using the same challenge protocol to evaluate the effect of immunization on survival time. In the first experiment (Fig. 4B), the 6 mice immunized with Ag38-transduced cells survived significantly longer than the 6 non-immunized control mice (p=0.0005), which all developed lung metastases and died within 31 days after tumor inoculation. Two out the 6 immunized mice were still alive at the end of the observation period (90 days). In the second experiment, a significant increase in survival time (p=0.0001)
was observed in the 8 immunized mice versus the 8 control mice, and 2 animals were still alive after 150 days. These data indicate that vaccination with murine melanoma cells transduced with the 38-kDa Mycobacterium gene induces a significant inhibition of lung metastases of i.v.-injected parental tumors, leading to prolonged survival.

### Th1 and Th2-type cytokine secretion pattern

Based on increasing evidence that suggests the importance of the Th1-type response in the control of tumor growth, we evaluated the Th1- and Th2-types of reactions in mice injected in the footpad with i) irradiated wild-type melanoma cells, ii) irradiated transduced cells, and iii) irradiated wild-type melanoma cell plus the 38-kDa purified protein. As reported in Table 1, analysis of cytokine levels in the culture supernatants of lymphocytes obtained from ipsilateral popliteal lymph nodes revealed an increase in IFN-γ levels in mice injected with transduced cells compared to mice injected with irradiated wild-type melanoma cell and to mice injected with irradiated wild-type melanoma cell plus the 38-kDa purified. In contrast, secreted IL-4 levels were higher in mice treated with wild-type B16 plus purified 38-kDa protein than in mice injected with transduced cells. Th1 switch was further confirmed by the finding that IL-2 was detectable only in the supernatant of lymphocytes from animals injected with transduced cells (data not shown). The induction of a preferential Th1 cytokine secretion pattern by endogenously expressed mycobacterial antigen is consistent with the studies by Huygen et al. ¹³ and Zhu et al. ¹⁴, who showed that vaccinations with plasmid DNA constructs encoding the 85T or 38-kDa Mycobacterium tuberculosis antigens are a powerful approach to generating a specific helper response with a Th1-like phenotype.
As previously reported ¹⁵, the B78 variant of melanoma B16 expressed undetectable levels of major histocompatibility complex (MHC) class I antigens in vitro, even after exposure to IFN-γ (not shown). Low expression of class I antigens frequently characterizes advanced tumors and metastases in humans.¹⁶ Indeed, down regulation of MHC class I expression is an important mechanism by which tumors may evade T cell-mediated immune responses. The expression of the 38-kDa protein on the B16-B78 melanoma cell surface might stimulate MHC-non-restricted cells, such as γ/δ T cells, which in turn influence the initial activation of Th1-cell subset.
Irrespective of MHC context, a recent report suggests that immunologic intervention with molecules that selectively activate Th1 responses may be effective in activating NK cells, cytotoxic T lymphocytes, and tumoricidal macrophages, thus increasing the killing of tumor cells.¹⁷
Enhancement of the immune response against tumor antigens has been achieved by transducing tumor cells with two different genes encoding heat-shock protein (HSP) 65 from Mycobacterium leprae ¹⁸ and Mycobacterium bovis.¹⁹ HSPs are molecular chaperones that mediate the assembly and folding of other proteins.²⁰ HSPs are also associated in vivo with the entire repertoire of peptides generated within the cell, and these non-covalent HSP-peptide complexes are particularly immunogenic.²¹ In the present study, we have shown that the expression of a Mycobacterium tuberculosis protein lacking chaperone activity ²² can enhance the immunogenicity of weakly antigenic tumor cells defective in co-stimulatory molecules and not expressing detectable levels of MHC antigens.
This transduction approach may offer a promising immunotherapeutic strategy for the treatment of cancer.

A detailed description of the invention based on figures and experimental data follows.

### DESCRIPTION OF FIGURES

**Fig. 1**
   Schematic representation of the pLAg38TMSN retroviral vector. LTR=Moloney murine leukemia virus long terminal repeat; Ψ=Packaging signal; Ag38=Mycobacterium tuberculosis Ag38 gene; SV=SV40 early promoter; NEO=neomycin phosphotransferase gene. Arrows indicate the transcription start. Not drawn to scale.
**Fig. 2**
   Expression of Ag38 in transduced cells. A) Detection of Ag38 mRNA by RT-PCR of total RNA from: B16-B78 wild-type melanoma cells (lane 2); B16-B78 cells transduced with pLTMSN vector (lane 3); B16-B78 cells transduced with pLAg38TMSN vector (lane 4); and pLAg38TMSN vector (lane 5). Lane 1, molecular weight marker (1 kb ladder). B) Detection of Ag38 protein by Western blot analysis of membrane proteins extracted from: B16-B78 wild-type melanoma cells (lane 2); B16-B78 cells transduced with pLTMSN vector (lane 3); and B16-B78 cells transduced with pLAg38TMSN vector (lane 4). Lane 1, bacterial recombinant Ag38 protein. C) Detection of Ag38 protein by FACScan analysis on the surface of B16-B78 cells transduced with pLTMSN vector (top) and B16-B78 cells transduced with pLAg38TMSN vector (bottom). Open areas indicate cells stained with secondary antibody alone.
**Fig. 3**
   Percent tumor incidence (A) and overall survival (B) of mice either non-immunized (dotted line) or immunized with 10⁶ irradiated wild-type B16-B78 (dashed line) or with irradiated transduced B16-B78 melanoma cells (solid line). Mice were challenged with 2.5x10⁵ wild-type B16-B78 melanoma cells.
**Fig. 4**
   Lung colonization and overall survival of mice challenged i.v. with 5x10⁵ wild-type B16-B78 melanoma cells. A) Lung metastases from mice immunized with irradiated transduced B16-B78 melanoma cells (upper) and from non-immunized mice (bottom). B) Survival of immunized mice (dashed line) and of non- immunized mice (solid line).

### EXAMPLES

### Retroviral construct

A SacII-PvuII fragment containing the first 843 bp of the nerve growth factor receptor (NGFR) lacking the intracellular domain was cloned into pGEM 5Zf+ (Promega); the extracellular portion of the NGFR was excised with StuI and AvaI and replaced in-frame with a synthetic polynucleotide sequence containing three unique cloning sites (NotI, ClaI and EcoRV) (designated pLTM). At the 3'-end, the last 28 bp of the human IL-3 sequence, containing the stop codon, were joined in-frame by replacement of the NcoI-NheI IL-3 fragment with the SacII-SpeI fragment of pLTM in the pBBG14 vector (British Biotechnology; Oxon, UK). The entire construct was excised from the plasmid (HindIII-BamHI) and ligated into the HpaI-BamHI sites of the retroviral vector pLXSN (courtesy of Dr. D. Miller; Fred Hutchinson Center, Seattle, WA). The entire vector, designated pLTMSN, was sequenced using the Sanger method (Sequenase Version 2, USB Amersham Life Science; Cleveland, OH).
DNA encoding residues 24-372 of Mycobacterium tuberculosis Ag38 was obtained by PCR using plasmid pMS9-2⁹ as template and two primers modified to include NotI and ClaI sites at the 5' and 3' ends, respectively. The sense primer was 5'-GCGGCCGCTGGCTCGAAACCACCGAGCGGTTCGCCT GAA-3', corresponding to nucleotides 215-246 of the Ag38 gene, and the antisense primer was 5'-GCGGTGGTGAAGTTGTCTGACGCGTTGATCGCATCGATT-3', corresponding to nucleotides 1235-1267. PCR was performed for 30 cycles of 1 min at 94°C, 1 min at 55°C and 2 min at 72°C. The PCR product was digested with NotI and ClaI restriction enzymes and ligated in-frame in the corresponding sites of the pLTMSN retroviral vector to obtain the pLAg38TMSN vector (Fig. 1).
Retroviral particles were obtained by infection techniques as described,²³ using the ampho-ecotropic packaging cell lines gp+AM12 and gp+E86.^{24,25} G418-resistant individual colonies of producer cells were screened for cell surface Ag38 expression by FACScan analysis using anti-Ag38 MAb HBT12.⁹ Virus produced by the best packaging cell line, in terms of viral titer and antigen expression, were used to infect murine melanoma cells.

### Mycobacterium gene transduction and expression in cells

B16-B78 murine melanoma cells¹¹ (6×10⁵) were cultured for 2 h with 5 ml of undiluted viral supernatant from the packaging cell line and 8 µg/ml of Polybrene; fresh medium was then added to dilute the supernatant (1:2) for the following 24 h. To improve infection efficiency, the same infection cycle was repeated 5 times. Individual colonies as well as a bulk cultures of G418-selected (1 mg/ml) melanoma cells were tested for 38-kDa antigen gene expression by RT-PCR, FACScan analysis and Western blotting.
G418-resistant colonies recovered from B16-B78 melanoma cells transfected with the empty vector were used as controls. For RT-PCR, total RNA was obtained from 1×10⁷ cells (Rneasy® Handbook, QIAGEN GmbH; Hilden, Germany); 1 µg of the RNA was reverse-transcribed (GeneAmp Rna PCR Kit, Perkin-Elmer Cetus; Norwalk, CT) and the cDNA was amplified by PCR utilizing the same 5' primer used for cloning and a 3' primer (5'-TGATCGCCCAGTGCAGAAAT-3') corresponding to nucleotides 1265-1284. Thirty cycles of amplification (1 min at 94°C, 1 min at 55°C and 2 min at 72°C) were performed and the reaction product (950 bp) was electrophoresed and stained with ethidium bromide.
Flow cytometry was used to detect cell surface antigen. Briefly, cells were incubated for 30 min at 4°C with 10 µg/ml of the anti-Ag38 purified MAb HBT12, washed and incubated for an additional 30 min at 4°C with goat anti-mouse IgG+IgM(H+L) FITC-conjugated secondary antibody (Kirkegaard & Perry Laboratories Inc.; Gaithersburg, MD). After washing, cells were analyzed by FACScan (Becton Dickinson, Mountain View, CA).
For Western blot analysis, membrane protein extract was obtained according to Landowsky et al.²⁶ with minor modifications: 1.5×10⁸ cells were lysed in ice-cold 25 mM Tris/0.3 M sucrose (pH 7.4) buffer, including protease inhibitor, by sonication with 5×5-s bursts on ice, followed by centrifugation at 500g for 10 min at 4°C. This step was repeated 3 times and the supernatant was collected. The membrane fraction was recovered by centrifugation of the supernatant at 143,000g for 90 min at 4°C. The pellet was resuspended and rotated end-over-end for 12-16 h at 4°C in 50 mM Tris/150 mM NaCl, pH 7.4, buffer containing 1% NP40 detergent and protease inhibitor. Insoluble material was pelleted by centrifugation at 200,000g for 60 min and the supernatant was electrophoresed on a 15% reducing SDS-PAGE gel. Proteins were detected using MAb HBT12 as primary antibody and visualized with peroxidase-coupled secondary antibody using the ECL detection system (Amersham).

### In vivo experiments

C57BL/6 female mice, 6 to 8 weeks old, were purchased from Charles River (Calco, Italy). All mice were handled and treated in accordance with institutional guidelines.
Tumorigenicity of cells was tested in mice injected subcutaneously (s.c.) with 2.5×10⁵ transduced or parental tumor cells in the right flank. For immunization, 10⁶ transduced or parental irradiated (20,000 rad) melanoma cells were injected s.c. (twice with a 4-week interval) in the left flank of 20 mice (10 animals per group). Four weeks after the second immunization, mice were challenged s.c. in the right flank with 2.5×10⁵ wild-type melanoma cells. Nine control mice received only the challenge with viable cells. Mice were checked for tumors twice weekly and two perpendicular diameters of tumor masses were recorded.
In experiments to test a vaccination protocol against experimental lung metastases, mice were immunized with irradiated cells as described above followed by i.v. inoculation of 5×10⁵ viable parental melanoma cells. Control mice received only the i.v. inoculation. Mice were either sacrified 4 weeks later to determine the number of lung metastasis. The same experimental protocol was used to evaluate the survival time in two experiments performed with 6 and 8 animals per group, respectively. Differences between groups were analyzed using the log rank test.
In experiments to evaluate the patterns of cytokine release by T cells, mice (6 per group) were immunized s.c. into the right hind footpad with 5 ×10⁶ untransduced or transduced irradiated cells, or with 3 µg Ag38 purified protein mixed with the untransduced irradiated cells, in 50 µl of saline. Five days later, mice were sacrified and popliteal lymph nodes were aseptically removed. Pools for each group were made, each containing the nodes of 2 or 3 animals. Lymphocytes were mechanically dissociated and cultured (2×10⁵ cells per well) in 96-well flat-bottom plates precoated or not with 1 µg/well of anti-CD3 MAb at 37°C for 18 h. Cytokine-containing supernatant was collected and tested for IFN-γ and IL-4 production by ELISA commercial kits (Genzyme; Cambridge, MA, USA). Unpaired t test was used to evaluate the significance of differences for the IL-4/IFN-γ ratio between groups.

**Table 1.**

| Secretion of, IL-4 and IFN-γ in supernatants from lymphocytes of melanoma-injected mice. | | |
|---|---|---|
| Injected antigen | Cytokine production (pg/ml)* | |
| | IL-4 | IFN-γ |
| B16 | 5.6±0.7 | 8.2±1.1 |
| B16 transduced | <5 | 32±7.5 |
| B16+Ag38 protein | 13.6±2 | <5 |

| | | |
|---|---|---|
| *Data refer to a representative experiment performed in triplicate and are expressed as mean±SD obtained subtracting the cytokine levels in the supernatant of lymphocytes cultured in the absence of anti-CD3 antibody. IL-4/IFN-γ ratio between: B16 transduced to B16 p=0.0013 ; B16 transduced to B16+Ag38 p=0.0109 (Unpaired t test). | | |

### Additional Example

To determine whether the Mycobacterium tuberculosis protein can also enhance immune recognition of other antigens not restricted to melanoma cells, we studied another antigenic model.

This model consists in mice transgenic for the rat neu proto-oncogene inserted in the germ line under the MMTV promoter. These transgenic mice develop spontaneous mammary carcinomas after a latency period over 6 months, which overexpress the oncoprotein encoded by the transgene.

The c-erbB-2 human oncogene homologue of the rat neu gene has been shown to be immunogenic in patients; therefore we selected this modul to study whether transduction of the Mycobacterium tuberculosis protein can enhance anti-neu immunogenicity.

To obtain the endogenous expression of the Mycobacterium tuberculosis protein, we transduced in vitro with the retroviral vector pLAg38TMSN the N202-1A tumor cell line, which is a cell line derived from a transgenic mouse mammary carcinoma, characterized by the overexpression of the neu oncoprotein.

We selected a clone expressing adequate levels of mycobacterial antigen on the cell surface and used this clone to perform some in vivo experiments.

To evaluate the effect of in vivo vaccination with transduced cells in inducing a protection against spontaneous tumor development, we immunized female mice twice with irradiated transduced or not-transduced cells 2-4 months before the spontaneous tumor arises and we evaluated and compared between groups the growth of spontaneous focal mammary tumors.

In a preliminary experiment with a small number of animals (5 for each group), we observed that all mice immunized with parental cells developed spontaneous tumor, while a partial protection was observed in 2 out of 5 mice immunized with transduced cells (a delay in tumor development in 1 animal and absence of tumor in the other animal). The result was not significant due to the small number of the group.

The same protocol was used to immunize a larger group of animals (11 for each group) in a subsequent experiment. We observed tumor development in all animals in both groups, with a delay in 7 out of 11 mice immunized with transduced cells. Moreover mice immunized with transduced cells displayed the development of smaller and lower number of tumors.

### REFERENCES

1 Pardoll DM. Cancer vaccines: a road map for the next decade. *Curr Opin Immunol* 1996; **8**:619-621.
2 Grange JM, Stanford JL, Rook GAW. Tuberculosis and cancer: parallels in host responses and therapeutic approaches? *Lancet* 1995; **345**:1350-1352.
3 Grange JM, Stanford JL. BCG vaccination and cancer. *Tubercle* 1990; **71**:61-64.
4 Musiani P *et al.* Cytokines, tumor-cell death and immunogenicity: a question of choice. *Immunol Today* 1997; **18:**32-36.
5 Bishop JM. Retroviruses and oncogenes II (Nobel lecture). *Angewandte Chemie-International Edition* 1990; **29**:716-724.
6 Chirivi RGS *et al.* IL-1a gene-transfected human melanoma cells increase tumor-cell adhesion to endothelial cells and their retention in the lung of nude mice. *Int J Cancer* 1996; **67:**856-863.
7 Kim TS, Russell SJ, Collins MKL, Cohen EP. Immunity to B16 melanoma in mice immunized with IL-2-secreting allogenic mouse fibroblasts expressing melanoma-associated antigens. *Int J Cancer* 1992; **51**:283-289.
8 Matzinger P. Tolerance, danger, and the extended family. *Annu Rev Immunol* 1994; **12**:991-1045.
9 Singh M *et al.* The mycobacterium tuberculosis 38-kDa antigen: overproduction in escherichia coli, purification and characterization. *Gene* 1992; **117**:55-60.
10 Vordermeier HM *et al.* M. tuperculosis-complex specific T-cell stimulation and DTH reactions induced with a peptide from the 38-kDa protein. *Scand J Immunol* 1992; **35**:711-718.
11 Graf L, Kaplan P, Silagi S. Efficient DNA-mediated transfer of selectable genes and unselected sequences into differentiated and undifferentiated mouse melanoma clones. *Somatic Cell Mol Genet* 1984; **10**:139
12 Poste G, Fidler IJ. The pathogenesis of cancer metastasis. *Nature* 1980; **283**:139-146.
13 Huygen K *et al.* Immunogenicity and protective efficacy of a tuberculosis DNA vaccine. *Nature Med* 1996; **2**:893-898.
14 Zhu X *et al.* Functions and specificity of T cells following nucleic acid vaccination of mice against mycobacterium tuberculosis infection. *J Immunol* 1997; **158:**5921-5926.
15 Lollini P *et al.* Enhancement of experimental metastatic ability by tumor necrosis factor-alpha alone or in combination with interferon-gamma. *Clin Exp Metastasis* 1990; **8**:215
16 Garrido F, Cabrera T, Lopez-Nevot MA, Ruiz-Cabello F. HLA class I antigens in human tumors. *Adv Cancer Res* 1995; **67**:155-195.
17 Sredni B *et al.* Predominance of TH1 response in tumor-bearing mice and cancer patients treated with AS101. *J Natl Cancer Inst* 1996; **88**:1276-1284.
18 Lukacs KV, Lowrie DB, Stokes RW, Colston MJ. Tumor cells transfected with a bacterial heat-shock gene lose tumorigenicity and induce protection against tumors. *J Exp Med* 1993; **178**:343-348.
19 Schweighoffer T. Tumor cells expressing a recall antigen are powerful cancer vaccines. *Eur J Immunol* 1996; **26**:2559-2564.
20 Hartl FU. Molecular chaperones in cellular protein folding. *Nature* 1996; **381**:571-580.
21 Blachere NE, Srivastava PK. Heat shock protein-based cancer vaccines and related thoughts on immunogenicity of human tumors. *Semin Cancer Biol* 1995; **6**:349-355.
22 Young DB, Garbe TR. Lipoprotein antigens of Mycobacterium tubercolosis. *Res Microbiol* 1991; **142**:55-65.
23 Colombo MP *et al.* Granulocyte colony-stimulating factor gene transfer suppresses tumorigenicity of a murine adenocarcinoma in vivo. *J Exp Med* 1991; **173**:889-897.
24 Markowitz D, Goff S, Bank A. Construction and use of a safe and efficient amphotropic packaging cell line. *Virology* 1988; **167:**400-406.
25 Markowitz D, Goff S, Bank A. A safe packaging line for gene transfer: separating viral genes on two different plasmids. *J Virol* 1988; **62**:1120-1124.
26 Landowski TH, Dratz EA, Starkey JR. Studies of the structure of the metastasis-associated 67 kDa laminin binding protein: fatty acid acylation and evidence supporting dimerization of the 22 kda gene product to form the mature protein. *Biochemistry* 1995; **34:** 11276-11287.
27 Zhu X *et al*. Specificity of CD8⁺ T cells for subunit vaccinated and infected H-2^{b}mice recognizing the 38kda antigen of Mycobacterium tuberculosis. *International Immunology* 1997; **9:** 1669-1676.
28 Jones VE, Mitchell MS. Therpeutic vaccines for melanoma: progress and problems. *Trends in Biotechnology* 1996; **14:** 349-355.

## Claims

1. Use of an expression vector for the preparation of a pharmaceutical for the treatment of neoplasms which vector comprises nucleic acid including at least one sequence which encodes in expressible form a polypeptide which is
(i) an antigen known as 38 kDa antigen of *Mycobacterium tuberculosis* or
(ii) a 33 kDa protein being a N-terminal deletion derivative of (i), wherein the protein does not contain the signal sequence, or contains only 17 to 24 codons of the signal sequence, or does not contain the signal sequence and additional 24 amino acids at the N-terminus.

2. The use according to claim 1 wherein the expression vector is a virus.

3. The use according to claim 2 wherein the nucleic acid is RNA.

4. The use according to claim 3 wherein the expression vector is a disabled retro virus.

5. The use according to claim 3 wherein the expression vector is a retroviral shuttle vector.

6. The use according to claim 5 wherein the expression vector is pLTMSN as shown in figure 1.

7. The use according to claim 2 wherein the expression vector is a vaccinia virus.

8. The use according to claim 2 wherein the expression vector is an adenovirus.

9. The use according to claim 1 wherein the expression vector is a plasmid.

10. The use according to claim 1 wherein the expression vector is an episomal vector.

11. The use according to any one of the preceding claims which further includes a sequence which encodes a neoplasms associated antigen.

12. The use according to any one of the preceding claims wherein the expression vector further includes a sequence which encodes an interleukin.

13. The use of a cell which includes an expression vector as claimed in any one of the preceding claims for the preparation of a pharmaceutical for the treatment of neoplasm.

14. The use of a neoplasm cell line the cells of which include an expression vector as claimed in any one of claims 1 to 12 for the preparation of a pharmaceutical for the treatment of neoplasm.

15. The use according to claim 13 or 14 wherein the cell is a human cell or cell line.

16. The use according to any one of claims 13 to 15 wherein the cell or cell line further includes an expression vector having a sequence which encodes a neoplasm associated antigen.

17. The use according to any one of claims 13 to 15 wherein the cell or cell line further includes an expression vector having a sequence which encodes an interleukin.

18. The use wherein the pharmaceutical as claimed in any one of the preceding claims additionally comprises a pharmaceutical acceptable carrier.

19. The use according to claim 18 wherein the carrier is liposome.

20. The use according to claim 18 or 19 wherein the pharmaceutical further comprises an expression vector having a sequence which encodes a neoplasm associated antigen or an interleukin.

21. Use of nucleic acid as defined in claim 1 for the manufacture of a medicament for the treatment of neoplasms.

22. Use according to any one of the preceding claims wherein the treatment is of existing neoplasms.

## Patentansprüche

1. Verwendung eines Expressionsvektors zur Herstellung eines Pharmazeutikums zur Behandlung von Neoplasmen, wobei der Vektor Nukleinsäure umfasst, einschließlich mindestens einer Sequenz, die in exprimierbarer Form ein Polypeptid kodiert, dieses ist
i) ein als 38 kDa Antigen von *Mycobacterium tuberculosis* bekanntes Antigen oder
ii) ein 33 kDa Protein, das ein Derivat von i) mit N-terminaler Deletion ist, wobei das Protein nicht die Signalsequenz enthält, oder nur 17 bis 24 Kodons der Signalsequenz enthält, oder nicht die Signalsequenz und weitere 24 Aminosäuren am N-Terminus enthält.

2. Verwendung gemäß Anspruch 1, wobei der Expressionsvektor ein Virus ist.

3. Verwendung gemäß Anspruch 2, wobei die Nukleinsäure RNA ist.

4. Verwendung gemäß Anspruch 3, wobei der Expressionsvektor ein inaktiviertes Retrovirus ist.

5. Verwendung gemäß Anspruch 3, wobei der Expressionsvektor ein retroviraler Shuttlevektor ist.

6. Verwendung gemäß Anspruch 5, wobei der Expressionsvektor pLTMSN, wie in Figur 1 gezeigt, ist.

7. Verwendung gemäß Anspruch 2, wobei der Expressionsvektor ein Vacciniavirus ist.

8. Verwendung gemäß Anspruch 2, wobei der Expressionsvektor ein Adenovirus ist.

9. Verwendung gemäß Anspruch 1, wobei der Expressionsvektor ein Plasmid ist.

10. Verwendung gemäß Anspruch 1, wobei der Expressionsvektor ein episomaler Vektor ist.

11. Verwendung gemäß einem der vorherigen Ansprüche, weiterhin enthaltend eine Sequenz, die ein mit Neoplasmen assoziiertes Antigen kodiert.

12. Verwendung gemäß einem der vorherigen Ansprüche, wobei der Expressionsvektor weiterhin eine Sequenz, deren Interleukin kodiert, enthält.

13. Verwendung einer Zelle, die einen Expressionsvektor gemäß einem der vorherigen Ansprüche enthält, zur Herstellung eines Pharmazeutikums zur Behandlung von Neoplasmen.

14. Verwendung einer neoplastischen Zelllinie, wobei deren Zellen einen Expressionsvektor gemäß einem der Ansprüche 1 bis 12 enthält, zur Herstellung eines Pharmazeutikums zur Behandlung von Neoplasmen.

15. Verwendung gemäß Anspruch 13 oder 14, wobei die Zelle eine normale Zelle oder Zelllinie ist.

16. Verwendung gemäß einem der Ansprüche 13 bis 15, wobei die Zelle oder Zelllinie weiterhin einen Expressionsvektor mit einer Sequenz, die für ein mit Neoplasmen assoziiertes Antigen kodiert, enthält.

17. Verwendung gemäß einem der Ansprüche 13 bis 15, wobei die Zelle oder Zelllinie weiterhin einen Expressionsvektor mit einer Sequenz, die für ein Interleukin kodiert, enthält.

18. Verwendung, wobei das gemäß einem der vorherigen Ansprüche beanspruchte Pharmazeutikum weiterhin einen pharmazeutisch annehmbaren Träger enthält.

19. Verwendung gemäß Anspruch 18, wobei der Träger ein Liposom ist.

20. Verwendung gemäß Anspruch 18 oder 19, wobei das Pharmazeutikum weiterhin einen Expressionsvektor mit einer Sequenz, die für ein Antigen, das mit Neoplasmen assoziiert ist, oder für ein Interleukin kodiert, enthält.

21. Verwendung einer Nukleinsäure gemäß Anspruch 1 zur Herstellung eines Medikaments zur Behandlung von Neoplasmen.

22. Verwendung gemäß einem der vorherigen Ansprüche, wobei die Behandlung auf vorhandene Neoplasmen gerichtet ist.

## Revendications

1. Utilisation d'un vecteur d'expression pour la préparation d'un produit pharmaceutique pour le traitement de néoplasmes dont le vecteur comprend un acide nucléique incluant au moins une séquence qui encode sous une forme exprimable un polypeptide qui est
(i) un antigène connu comme étant l'antigène 38 kDa de *Mycobacterium tuberculosis* ou
(ii) une protéine 33 kDa qui est un dérivé de suppression de N-terminaux de (i), dans laquelle la protéine ne contient pas la séquence signal, ou contient seulement 17 à 24 codons de la séquence signal, ou ne contient pas la séquence signal et d'acides aminés additionnels 24 aux N-terminaisons.

2. L'utilisation selon la revendication 1 dans laquelle le vecteur d'expression est un virus.

3. L'utilisation selon la revendication 2 dans laquelle l'acide nucléique est l'ARN.

4. L'utilisation selon la revendication 3 dans laquelle le vecteur d'expression est un rétro virus de handicap.

5. L'utilisation selon la revendication 3 dans laquelle le vecteur d'expression est un vecteur rétroviral navette.

6. L'utilisation selon la revendication 5 dans laquelle le vecteur d'expression est le pLTMSN tel que montré à la figure 1.

7. L'utilisation selon la revendication 2 dans laquelle le vecteur d'expression est un virus vaccinia.

8. L'utilisation selon la revendication 2 dans laquelle le vecteur d'expression est un adénovirus.

9. L'utilisation selon la revendication 1 dans laquelle le vecteur d'expression est un plasmide.

10. L'utilisation selon la revendication 1 dans laquelle le vecteur d'expression est vecteur épisomal.

11. L'utilisation selon l'une quelconque des revendications précédentes incluant en outre une séquence qui encode un antigène associé aux néoplasmes.

12. L'utilisation selon l'une quelconque des revendications précédentes dans laquelle le vecteur d'expression comprend en outre une séquence qui encode une interleukine.

13. L'utilisation d'une cellule qui comprend un vecteur d'expression selon l'une quelconque des revendications précédentes pour la préparation d'un produit pharmaceutique pour le traitement de néoplasmes.

14. L'utilisation d'une lignée cellulaire de néoplasmes dont les cellules incluent un vecteur d'expression tel que revendiqué dans l'une quelconque des revendications 1 à 12 pour la préparation d'un produit pharmaceutique pour le traitement de néoplasmes.

15. L'utilisation selon la revendication 13 ou 14 dans laquelle la cellule est une cellule ou lignée cellulaire humaine.

16. L'utilisation selon l'une quelconque des revendications 13 à 15 dans laquelle la cellule ou lignée cellulaire comprend en outre un vecteur d'expression ayant une séquence qui encode un antigène associé aux néoplasmes.

17. L'utilisation selon l'une quelconque des revendications 13 à 15 dans laquelle la cellule ou lignée cellulaire comprend en outre un vecteur d'expression ayant une séquence qui encode une interleukine.

18. L'utilisation dans laquelle le produit pharmaceutique selon l'une quelconque des revendications précédentes comprend en outre un véhicule pharmaceutique acceptable.

19. L'utilisation selon la revendication 18 dans laquelle le véhicule est un liposome.

20. L'utilisation selon la revendication 18 ou 19 dans laquelle le produit pharmaceutique comprend en outre un vecteur d'expression ayant une séquence qui encode un antigène associé aux néoplasmes ou une interleukine.

21. Utilisation d'un acide nucléique telle que défini dans la revendication 1 pour la fabrication d'un médicament pour le traitement de néoplasmes.

22. Utilisation selon l'une quelconque des revendications précédentes dans laquelle le traitement est celui de néoplasmes existants.
